# EUROPÄISCHE PATENTANMELDUNG

(11) **EP 3 192 514 A1**
(43) Veröffentlichungstag der Anmeldung: **19.07.2017**
(21) Anmeldenummer: 16000071.7
(22) Anmeldetag: 12.01.2016
(51) Int. Cl.: A61K 31/575, A61K 47/10, A61K 47/44

(54) **PHARMAZEUTISCHE ZUSAMMENSETZUNG ENTHALTEND 7SS-HYDROXYCHOLESTEROL GEEIGNET ZUR INTRAVENÖSEN VERABREICHUNG**

(71) Anmelder: Immunopharm AG, 9496 Balzers (LI)
(72) Erfinder: Die Erfindernennung liegt noch nicht vor
(74) Vertreter: Schüssler, Andrea

(57) **Zusammenfassung**

Die vorliegende Erfindung betrifft eine pharmazeutische Zusammensetzung umfassend (a) 7β-Hydroxycholesterol, (b) Propylenglykol und/oder DMSO und (c) PEG hydriertes Rizinusöl und ein Verfahren zur Herstellung davon. Diese Zusammensetzung eignet sich zur Herstellung einer intravenös zu verabreichenden Zubereitung und umfasst die pharmazeutisch aktive Substanz 7β-Hydroxycholesterol, die in Wasser schlecht löslich ist.

## Beschreibung

### Gebiet der Erfindung

Die vorliegende Erfindung betrifft eine pharmazeutische Zusammensetzung umfassend (a) 7β-Hydroxycholesterol, (b) Propylenglykol und/oder DMSO und (c) PEG hydriertes Rizinusöl; und ein Verfahren zur Herstellung davon. Diese Zusammensetzung eignet sich zur Herstellung einer intravenös zu verabreichenden Zubereitung und umfasst die pharmazeutisch aktive Substanz 7β-Hydroxycholesterol, die in Wasser schlecht löslich ist.

### Stand der Technik

Zur Herstellung von pharmazeutischen Zusammensetzungen, um das pharmazeutisch aktive Mittel zu dispergieren, so dass die Zusammensetzung an einen Patienten verabreicht werden kann, wird ein geeignetes Lösungsmittel oder Trägersystem benötigt. Das Lösungsmittel muss in der Lage sein, eine therapeutisch wirksame Menge des Wirkstoffs zu lösen oder aufzulösen, um eine wirksame Zusammensetzung zu erzeugen. Jedoch sind viele pharmazeutisch wirksame Verbindungen nicht ausreichend löslich in Lösungsmitteln, wie z.B. Wasser. Ein weiteres Problem ist, dass zahlreiche pharmazeutische Wirkstoffe nach der Verdünnung für eine Infusionslösung instabil werden oder sie weisen eine Zersetzung oder einen Aktivitätsverlust des Wirkstoffes während der Lagerung in einem LösungsmittelSystem auf. Die geringe Löslichkeit und die Anfälligkeit für eine Zersetzung limitiert die Verwendung dieser schwer wasserlöslichen pharmazeutisch aktiven Substanzen in der Therapie erheblich.

Die meisten der derzeit verfügbaren Verabreichungsformen der pharmazeutisch aktiven Substanz 7β-Hydroxycholesterol sind Kapseln in oraler Form, da das 7β-Hydroxycholesterol nicht wasserlöslich ist.

Es gibt aber auch den Ansatz 7β-Hydroxycholesterol in wasserlöslicher Form herzustellen. Die DE 35 07 721 A1 beschreibt ein Verfahren zur Darstellung wasserlöslicher Derivate des 7β-Hydroxycholesterols. Um die Derivate wasserlöslich zu machen, wurde die Umsetzung derselben mit Bersteinsäureanhydrid durchgeführt und aus dem entstandenen Hemisuccinat wurde entweder das Natrium- oder das Kalium-Salz gebildet.

Christ et al. (Anticancer Res. 1991, 11(1):359-64) beschreiben die Herstellung von Phosphodiestern von 7β-Hydroxycholesterol. Diese beiden Salze haben eine anti-Tumor-Aktivität und sind zwar wasserlöslich, aber haben den Nachteil in wässriger Lösung nicht stabil zu bleiben.

Die EP 0 07 834 A1 betrifft wasserlösliche Derivate von Cholesterin, insbesondere wasserlösliche Derivate von 7-Hydroxycholesterol. Dies wird durch Verbindungen und Komplexen von Albumin mit organischen zweibasigen Säure-Halbestern von 7-Hydroxycholesterol erreicht.

Ein Nachteil des Standes der Technik ist, dass bisher die körpereigene Signalsubstanz 7β-Hydroxycholesterol nicht in wässriger Lösung eingesetzt werden konnte, sondern nur zwei wasserlösliche Salze derselben (Hydroxycholesterolbishemisuccinat-diethanolamin und 7β-Hydroxycholesterolbishemisuccinat-dinatrium). So beschreiben Maier et al. (Anticancer Res. 1999, 19(5b), S. 4251-4256) die Induktion von Apoptose durch beiden genannten Salze in menschlichen Kolonkarzinomzelllinien. Diese beiden Salze sind zwar wasserlöslich, aber in wässriger Lösung nicht stabil. Nach einigen Stunden beginnt die Substanz sich zu zersetzen und auszuflocken. Des Weiteren ist nicht bekannt, wie sich das Bishemisuccinat im Gegensatz zum 7β-Hydroxycholesterol verhält und welche zusätzlichen Nebenwirkungen von Ethanolamin bei der Anwendung im Menschen ausgehen können.

Ein weiterer Nachteil des Standes der Technik ist, dass 7β-Hydroxycholesterol in oraler Form nicht gerne verwendet wird, da nicht bekannt ist, wie viel von der Substanz am Zielort ankommt. Ebenfalls ist die Resorption des 7β-Hydroxycholesterol im Magen-Darm-Trakt nicht bekannt.

### Aufgabe der Erfindung

Daher besteht für eine gezielte pharmazeutische Anwendung der Bedarf, die Substanz 7β-Hydroxycholesterol in einer wässrigen Flüssigkeit in Lösung zu bringen bzw. zu halten und dann intravenös, z.B. per Infusion, verabreichen zu können.

### Ausführliche Beschreibung der Erfindung

Die vorliegende Erfindung löst das technische Problem durch die Bereitstellung einer Zusammensetzung, umfassend die pharmazeutisch aktive Substanz 7β-Hydroxycholesterol und ein Lösungsmittelsystem, das ein Lösungsmittel und einen Lösungsvermittler umfasst.

Erfindungsgemäß wird die Aufgabe durch eine pharmazeutische Zusammensetzung enthaltend
(a) 7β-Hydroxycholesterol
(b) Propylenglykol und/oder DMSO
(c) PEG hydriertes Rizinusöl
gelöst.

7β-Hydroxycholesterol (C₂₇H₄₆O₂) ist eine universell vorkommende Signalsubstanz mit einem Molekulargewicht von 402.65294 g/mol, die in der Thymusdrüse produziert wird und eine anti-proliferatorische Wirkung hat sowie eine universelle Signalsubstanz der körpereigenen Immunabwehr ist. Es wird daher vornehmlich zur Krebstherapie verschiedenster Tumoren und zur allgemeinen Steigerung der Immunabwehr eingesetzt. Es ist ein Stoffwechselprodukt, das durch Cholesterinoxidation entsteht. Als Oxysterole werden im Allgemeinen die sauerstoffhaltigen Abkömmlinge des Cholesterins (Cholesterol) bezeichnet. Die Sauerstoff-Funktionen (Hydroxy-Gruppen, Keto-Gruppen oder Epoxy-Gruppen) sind dabei an den Kohlenstoffatomen des Cholesterol-Grundgerüsts lokalisiert. 7β-Hydroxycholesterol ist durch die Strukturformel 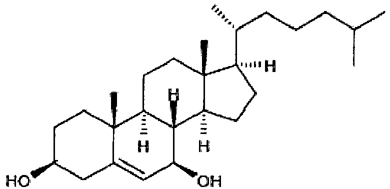 gekennzeichnet.

Der weitere Bestandteil Propylenglykol (1,2-Propandiol; C₃H₈O₂) ist als Lösungsmittel bekannt und wird erfindungsgemäß als solches für 7-Hydroxycholesterol verwendet. Es stellt eine klare, farblose, nahezu geruchlose und stark hygroskopische Flüssigkeit mit einem Molekulargewicht von 76,10 g/mol dar. Propylenglykol gehört zu den mehrwertigen Alkanolen. Es ist mit Wasser und Ethanol mischbar, mit fetten Ölen dagegen nicht. Bei hohen Temperaturen oberhalb 150 °C ist es oxidationsempfindlich. Propylenglykol ist eine chirale Verbindung (Stereozentrum an C2), die zumeist als Racemat eingesetzt wird. Vorteilhaft ist, dass Propylenglykol die Löslichkeit verschiedener Stoffe deutlich verbessert und eine stabilere Dispersion von Arzneistoffen gewährleisten kann. Darüber hinaus kann es häufig zu einer deutlichen Resorptionsverbesserung verschiedener Wirkstoffe beitragen. Die antimikrobielle Wirksamkeit macht einen Einsatz weiterer Konservierungsmittel häufig überflüssig. Desweiteren ist von Propylenglykol bekannt, dass es in Injektionsformulierungen vieler Arzneimittel enthalten ist, aber in bestimmten Konzentrationen lokale Unverträglichkeiten hervorrufen kann (siehe Kruss B. (Acta Pharm. Technol. 35(4) (1989) 187-196)). Es kann zu schmerzhaften Schwellungen und Gewebeunverträglichkeiten an der Injektionsstelle kommen, die sich zum Teil erst nach mehreren Wochen zurückbilden. Das Irritationspotential an der Haut ist in starkem Maße konzentrationsabhängig, ein geringer Zusatz wird aber allgemein als tolerierbar angesehen. Daher sollte der Einsatz in angemessener Konzentration erfolgen. Erfindungsgemäß wird in der zuzubereitenden Infusionslösung die die Irritationen hervorrufende kritische Konzentration (10-15 %) unterschritten und daher vom Patienten hervorragend vertragen. Propylenglykol ist durch die Strukturformel 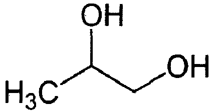 gekennzeichnet.

Alternativ oder zusätzlich zu Propylenglykol kann Dimethylsulfoxid (DMSO; C₂H₆OS) als Lösungsmittel verwendet werden. DMSO ist ein organisches Lösungsmittel und zählt zur Klasse der Sulfoxide. Es ist in jedem Verhältnis mit Wasser sowie vielen organischen Lösungsmitteln mischbar. DMSO selbst hat eine pharmazeutische Wirkung (antiphlogistisch und analgetisch) und wird oft in Salben, Gelen, Tinkturen oder Pflastern zur perkutanen Behandlung stumpfer Verletzungen und lokaler Schmerzzustände eingesetzt.

7β-Hydroxycholesterol weist in Propylenglykol und/oder DMSO eine hervorragende Löslichkeit auf und ermöglicht diese Lösung über Jahre stabil zu halten. Will man jedoch die 7β-Hydroxycholesterol/Propylenglykol- bzw. DMSO-Lösung in einer wässrigen Flüssigkeit, z.B. zur Bereitstellung einer Infusionslösung, verdünnen, flockt die Substanz aus. Von den Erfindern wird daher ein Lösungsvermittler eingesetzt, um das 7β-Hydroxycholesterol in der späteren Infusionslösung in Lösung zu halten.

Als besonders geeigneter Lösungsvermittler hat sich erfindungsgemäß ein PEG hydriertes Rizinusöl herausgestellt. Es konnte insbesondere gezeigt werden, dass durch die Verwendung von PEG-40 hydriertem Rizinusöl eine stabilisierte flüssige pharmazeutische Formulierung hergestellt werden konnte.

PEG hydriertes Rizinusöl ist ein Polyethylenglykol (PEG)-Derivat von hydriertem Rizinusöl und ist eine bernsteinfarbene, leicht viskose Flüssigkeit. Rizinusöl ist ein Pflanzenöl, das aus den Samen des tropischen Wunderbaums (*Ricinus communis*)*,* eines Wolfsmilchgewächses, gewonnen wird. Es besteht aus verschiedenen Triglyceriden und wird in der Pharmazie auch "Polyoxyethylated castor oil" genannt. Bei der Hydrierung des Rizinusöls wird an die Doppelbindung der Rizinolsäure Wasserstoff unter Verwendung von Raney-Nickel addiert. Die Hydroxygruppe bleibt erhalten und es entsteht ein Fett (Rizinuswachs), das Triglycerid der 12-Hydroxystearinsäure. Polyethylenglykole (PEG) sind ein synthetischer Rohstoff, dessen Basisbaustein das Ethylenoxid (C₂H₄O) ist. Mit Wasser entsteht aus Ethylenoxid das Ethylenglykol, das als Frostschutzmittel bekannt ist. Ethylenglykol kann mit Ethylenoxid weiterreagieren und dadurch Diethylenglykol und schließlich Polyethylenglykole (PEG) unterschiedlicher Länge mit entsprechend vielfältigen Eigenschaften bilden.

Handelsprodukte des PEG hydrierten Rizinusöls sind beispielsweise auf der Basis von PEG7, PEG25, PEG40, PEG50 und PEG60 bekannt: Croduret™ 25 (Fa. Croda), Croduret™ 40 (Fa. Croda), Croduret™ 50 (Fa. Croda), Croduret™ 60 (Fa. Croda) Cremophor® RH 40 (Fa. BASF), Cremophor® RH 410 (Fa. BASF), Emulgin® HRE 40 (Fa. BASF), Emulgin® HRE 455 (Fa. BASF) und EMAROL H40 (Fa. CISME, Italien). Erfindungsgemäß eignet sich PEG40 hydriertes Rizinusöl besonders.

Die Zusammensetzung kann vorzugsweise zusätzlich zu dem PEG hydrierten Rizinusöl weitere Lösungsvermittler enthalten, beispielsweise aus der Gruppe der Polyoxyethylene, wie polyethoxylierte Sorbitanfettsäureester, bevorzugt Polysorbat 80; polyethoxylierte Vitamin E Derivate, bevorzugt Vitamin E-Polyethylenglykol1000 Succiniat; Polyethoxylierte Fettsäurepartialglyceride, bevorzugt Diethylenglykolmonostearat; Polyethoxylierte Kohlenhydrate; Polymere von Ethylenoxid und Propylenoxid , bevorzugt Poloxamer 188.

Erfindungsgemäß enthält die pharmazeutische Zusammensetzung
a) 7β-Hydroxycholesterol 0,1-2,0 Gew.-%, bevorzugt 0,3-1,5 Gew.%, ganz bevorzugt 0,5-1,3 Gew.-%, insbesondere bevorzugt 0,2, 0,3, 0,4, 0,5, 0,6, 0,7, 0,8, 0,9, 1,0, 1,1, 1,2, 1,3, 1.4 oder 1,5 Gew.-%
b) Propylenglykol und/oder DMSO 68-97 Gew.-%, bevorzugt 70-95 Gew.-%, ganz bevorzugt 75-90 Gew.%, insbesondere bevorzugt 70, 71, 72, 73, 74, 75, 76, 77, 78, 80, 81, 82, 83, 84, 85, 86, 87, 88, 89, 90, 91, 92, 93, 94 oder 95 Gew.%
c) PEG hydriertes Rizinusöl 1-30 Gew.-%, bevorzugt 5-25 Gew.-%, ganz bevorzugt 5-20 Gew.-%, ganz besonders bevorzugt 5, 6, 7, 8, 9, 10, 11, 12, 13, 14, 15, 16, 17, 18, 19 oder 20 Gew-%.

Die Zusammensetzung kann weiter übliche pharmazeutisch verträgliche Zusätze enthalten, z.B. Alkohol (vorzugsweise 1-15 Gew-% Ethanol) oder Antioxydantien (vorzugsweise 1-10 Gew-% Caroverinhydrochlorid, 1-10 Gew-% Glutathion, 1-10 Gew.-% Vitamin C).

Bei der Herstellung von intravenös zu verabreichenden Zubereitungen, d.h. Infusionslösungen, sind dies bevorzugt wässrige Lösungen, wobei es möglich ist, diese gebrauchsfertig vorzuhalten oder direkt vor Gebrauch herzustellen.

Erfindungsgemäß kann die pharmazeutische Zusammensetzung mit einer wässrigen zur intravenösen Gabe geeigneten üblichen Flüssigkeit, bevorzugt physiologischer Kochsalzlösung, einer 5-10%igen Glukose-Lösung, Ringer-Lösung, Ringer-Laktat-Lösung und/oder HES zu einer Infusionslösung gemischt werden. Die entweder gebrauchsfertige Lösungen oder pharmazeutische Zusammensetzung (wenn die Infusionslösung erst direkt vor Gebrauch hergestellt wird) werden sterilisiert und sind anschließend bei Raumtemperaturen (< 25 °C) über Monaten haltbar. Die Sterilisierung erfolgt bevorzugt durch Sterilfiltration durch Filter sowie durch einen Autoklaven bei einer Temperatur von 120°C.

Nach einer Ausführungsform des erfindungsgemäßen Verfahrens wird eine entsprechende Menge 7β-Hydroxycholesterol in Propylenglykol und/oder DMSO durch leichtes Erwärmen bei 30-40 °C während 15 Minuten gelöst. Anschließend wird der Lösungsvermittler, vorzugsweise PEG-40 hydriertes Rizinusöl, dazugegeben und das entstandene Konzentrat gut gemischt. Dieses Konzentrat wird zur gewünschten Menge, bevorzugt 100 ml, 200 ml und 400 ml, wässriger Flüssigkeit, bevorzugt Glucoselösung oder physiologischer Kochsalzlösung, gegeben. Diese Lösung wird steril filtriert und anschließend bei über 100°C, vorzugsweise 120 °C -140°C, sterilisiert.

Nach einer weiteren Ausführungsform des erfindungsgemäßen Verfahrens wird eine entsprechende Menge 7β-Hydroxycholesterol in Propylenglykol und/oder DMSO durch leichtes Erwärmen bei 30-40 °C während 15 Minuten gelöst. Anschließend wird der Lösungsvermittler, vorzugsweise PEG-40 hydriertes Rizinusöl, dazugegeben und das entstandene Konzentrat gut gemischt. Dieses Konzentrat wird steril filtriert und anschließend bei über 100°C, vorzugsweise bei 120 °C -140°C, sterilisiert. Die erfindungsgemäße Zusammensetzung wird zu gegebener Zeit, z.B. kurz vor der Gabe als Infusion, mit der entsprechenden Menge steriler wässriger Flüssigkeit, z.B. steriler physiologischer Kochsalzlösung oder Glukoselösung gemischt, bevorzugt 100 ml, 200 ml oder 400 ml.

Im Rahmen der Erfindung konnte die Stabilität von 7β-Hydroxycholesterol in Propylenglykol und/oder DMSO sowie PEG 40 hydriertem Rizinusöl nachgewiesen werden. Die Lösung von 7β-Hydroxycholesterol in Propylenglykol und/oder DMSO sowie PEG 40 hydriertem Rizinusöl war problemlos mit physiologischer Kochsalzlösung und/oder einer Glukoselösung mischbar. Es konnte auch nachgewiesen werden, dass 7β-Hydroxycholesterol in der so hergestellten Infusionslösung über Wochen und Monate stabil war.

Die erfindungsgemäße Zusammensetzung wird insbesondere als Infusion eingesetzt zur Behandlung von Tumoren aller Phänotypen, zur Durchbrechung von Resistenzen bei der Behandlung von Krebs, zur Steigerung der allgemeinen Immunabwehr, zur Behandlung bakterieller und viraler Erkrankungen, Autoimmunerkrankungen sowie erhöhten Stress- und Umweltbelastungen. Die Verabreichungen erfolgt gemäß Standardverfahren zur intravenösen Verabreichung, insbesondere als Infusion.

Als besonders vorteilhaft hat sich erwiesen, die Zusammensetzung als Infusion in einem zeitlichen Abstand, z.B. 2-6 Stunden, vorteilhafterweise etwa 4 Stunden, zur Gabe von Caroverinkapseln (1-[2-(diethylamino)ethyl]- 3-(4-methoxybenzyl)quinoxalin- 2(1*H*)-on) oder Synoverin®-Kapseln (7β Hydroxycholesterol + Caroverin) zu verabreichen. Bevorzugt ist dabei die Gabe der erfindungsgemäßen Infusion ca. 4 Stunden vor der Caroverin- bzw. Synoverin®-Kapsel-Verabreichung.

### Beispiele

Die vorliegende Erfindung wird durch die nachfolgenden nicht einschränkenden Beispiele detailliert beschrieben.

### Beispiel 1:

| | |
|---|---|
| Ethanol 96 % | 0,3 g |
| Propylenglykol | 2,0 g |
| PEG40 hydriertes Rizinusöl | 0,5 g |
| 7β-Hydroxycholesterol | 0,01 g |
| Physiologische Kochsalzlösung | ad 100 ml |
| (oder 5%ige Glukoselösung) | |

### Beispiel 2:

| | |
|---|---|
| Propylenglykol | 20 g |
| PEG40 hydriertes Rizinusöl | 2 g |
| 7β-Hydroxycholesterol | 0,06 g |
| Physiologische Kochsalzlösung | ad 200 ml |
| (oder 5%ige Glukoselösung) | |

### Beispiel 3:

| | |
|---|---|
| Propylenglykol | 20 g |
| PEG40 hydriertes Rizinusöl | 2 g |
| 7β-Hydroxycholesterol | 0,04 g |
| Physiologische Kochsalzlösung | ad 400 ml |
| (oder 5%ige Glukoselösung) | |

### Beispiel 4:

| | |
|---|---|
| Propylenglykol | 7 g |
| PEG40 hydriertes Rizinusöl | 1,5 g |
| 7β-Hydroxycholesterol | 0,04 g |
| Physiologische Kochsalzlösung | ad 200 ml |
| (oder 5%ige Glukoselösung) | |

### Beispiel 5:

| | |
|---|---|
| Propylenglykol | 20 g |
| PEG40 hydriertes Rizinusöl | 1 g |
| 7β-Hydroxycholesterol | 0,04 g |
| Physiologische Kochsalzlösung | ad 50 ml |

### Beispiel 6:

| | |
|---|---|
| Dimethylsulfoxid (DMSO) | 20 g |
| PEG40 hydriertes Rizinusöl | 1 g |
| 7β-Hydroxycholesterol | 0,04 g |
| Physiologische Kochsalzlösung | ad 50 ml |

### Beispiel 7:

| | |
|---|---|
| Dimethylsulfoxid (DMSO) | 10 g |
| Propylenglykol | 10 g |
| PEG40 hydriertes Rizinusöl | 1 g |
| 7β-Hydroxycholesterol | 0,04 g |
| Physiologische Kochsalzlösung | ad 50 ml |

### Beispiel 8:

### Stabilitätsuntersuchungen:

Nach der Sterilisation bei 120 °C konnte weder beim Konzentrat noch bei der fertig zubereiteten Infusionslösung (physiologische Kochsalzlösung) eine Zersetzung des 7β-Hydroxycholesterols festgestellt werden. Das bedeutet, dass selbst bei dieser hohen thermischen Belastung das 7β-Hydroxycholesterol stabil blieb. Eine Lagerung des Konzentrats bei 40 °C während 3 Monaten zeigte ebenfalls keine Zersetzung des 7β-Hydroxycholesterols. Die Lösung wurde in Glasampullen gelagert.

Eine fertig zubereitete Infusionslösung (auf Basis von physiologischer Kochsalzlösung) wurde bei Raumtemperatur (< 25 °C) während 3 Monaten gelagert. Die Lösung blieb klar, es gab keine Ausflockung. Mittels HPLC konnte keine Abnahme des Gehalts an 7β-Hydroxycholesterol nach der Lagerung festgestellt werden.

## Patentansprüche

1. Pharmazeutische Zusammensetzung enthaltend (a) 7β-Hydroxycholesterol, (b) Propylenglykol und/oder DMSO und (c) PEG-hydriertes Rizinusöl.

2. Pharmazeutische Zusammensetzung nach Anspruch 1, **dadurch gekennzeichnet, dass** sie folgende Zusammensetzung aufweist:
(a) 7β-Hydroxycholesterol 0,1-2,0 Gew.-%
(b) Propylenglykol und/oder DMSO 68-97 Gew.-%
(c) PEG hydriertes Rizinusöl 1-30 Gew.-%

3. Pharmazeutische Zusammensetzung nach Anspruch 1 oder 2, wobei sie weiter einen oder mehrere Alkohole und/oder Antioxidantien aufweist.

4. Pharmazeutische Zusammensetzung nach einem der Ansprüche 1-3, wobei das PEG hydrierte Rizinusöl PEG40 hydriertes Rizinusöl ist.

5. Verfahren zur Herstellung einer pharmazeutischen Zusammensetzung nach Anspruch 1, umfassend das Lösen von 7β-Hydroxycholesterol durch leichtes Erwärmen in Propylenglykol und/oder DSMO und Hinzugeben des Lösungsvermittlers PEG hydriertes Rizinusöl.

6. Verfahren nach Anspruch 5, wobei die pharmazeutische Zusammensetzung steril filtriert und bei 120°C -140°C sterilisiert wird.

7. Verwendung der pharmazeutischen Zusammensetzung nach einem der Ansprüche 1-4 zur Herstellung einer intravenös zu verabreichenden Zubereitung.

8. Verwendung nach Anspruch 7, wobei die intravenös zu verabreichende Zubereitung eine Infusionslösung ist.

9. Verwendung nach Anspruch 8, wobei die Infusionslösung die pharmazeutische Zusammensetzung nach einem der Ansprüche 1-4 und eine wässrige Flüssigkeit enthält.

10. Verwendung nach Anspruch 9, wobei die wässrige Flüssigkeit physiologische Kochsalzlösung, Glucoselösung oder Ringer-Lösung ist.
